# FASCICULE DE BREVET EUROPEEN

(11) **EP 0 727 408 B1**
(45) Date de publication et mention de la délivrance du brevet: **09.12.1998**
(21) Numéro de dépôt: 96400303.2
(22) Date de dépôt: 14.02.1996
(51) Int. Cl.: C07C 67/08, C07C 69/14, C07C 67/54

(54) **Procédé de valorisation, sous la forme d'un ester, de l'acide acétique contenu dans les fractions légères de purification de l'acide acrylique**
Verfahren zur Verwertung, in Form eines Esters, von Essigsäure, welche in den leichten Fraktionen der Reinigung von Acrylsäure enthalten ist
Process for valorizing, in ester form, of acetic acid, contamid in the light fractions of the purification of acrylic acid

(30) Priorité: 17.02.1995 FR 9501869
(43) Date de publication de la demande: 21.08.1996
(73) Titulaire: ELF ATOCHEM S.A., 92800 Puteaux, Hauts-de-Seine (FR)
(72) Inventeur: Fauconet, Michel, 57730 Valmont (FR); Richard, Norbert, 57500 Saint Avold (FR)
(74) Mandataire: Rieux, Michel

(56) Documents cités:
- Aucun document pertinent relevé

## Description

La présente invention porte sur un procédé de valorisation, sous forme d'ester, de l'acide acétique contenu dans les fractions légères de purification de l'acide acrylique.

Dans les procédés de synthèse de l'acide acrylique, utilisant par exemple l'oxydation catalytique du propylène, l'une des impuretés majoritaires formées par réaction secondaire est l'acide acétique. Cette impureté, qui présente une température d'ébullition plus faible que l'acide acrylique, est généralement éliminée en tête d'une colonne de distillation, permettant de débarrasser le monomère de toutes ses impuretés légères. Le flux récupéré de cette manière est un mélange riche en acide acétique, mais il contient d'autres impuretés légères, comme l'eau, l'acide formique (réaction secondaire), l'acroléine (intermédiaire de la première étape d'oxydation du propylène), l'acide acrylique, et, éventuellement, des traces du solvant utilisé lors d'une étape précédente de récupération de l'acide acrylique à partir de ses solutions aqueuses.

Cet effluent très impur doit être éliminé, ce qui est effectué en général par incinération. Cette opération, qui permet une récupération très limitée d'énergie, représente une perte importante de matières nobles, en particulier d'acide acétique. En outre, l'acide formique présent dans le flux, connu pour ses fortes propriétés corrosives sur les métaux, entraîne des problèmes de corrosion au niveau de l'installation.

Outre ce phénomène de corrosion, la valorisation de ce mélange sous forme de ses constituants nobles se heurte à une difficulté de séparation de ces différents composés, du fait des faibles écarts entre leurs points d'ébullition et des associations qui existent entre ces produits sous forme de mélanges azéotropiques.

Pour valoriser cet effluent perdu, la Société déposante a imaginé de transformer ses constituants principaux en esters.

La synthèse des esters acétiques par réaction d'estérification de l'acide acétique par un alcool est bien décrite dans la littérature. Par exemple, l'article de Faith, Keyes et Clark dans "Industrial Chemicals", Ed. John Wiley & Sons, Inc., New York, 3^{ème} édition, page 176, décrit un procédé de synthèse classique d'acétate de butyle par estérification de l'acide acétique par le butanol, en présence d'acide sulfurique comme catalyseur. De même, la synthèse des esters acryliques par estérification est abondamment décrite dans la littérature.

Lorsqu'on réalise la réaction d'estérification à partir d'un mélange réel issu de la fraction d'étêtage d'un procédé de purification de l'acide acrylique, on obtient un mélange des différents constituants présents dans le mélange initial et n'ayant pas réagi et de leurs produits de réaction. Toutefois, il est apparu, de manière surprenante, que la séparation des produits de la réaction d'estérification, en vue d'extraire l'ester acétique, avec une pureté suffisante, est réalisable par un procédé beaucoup plus simple que la séparation des constituants du mélange initial.

C'est ce qui fait l'objet de la présente invention. Celle-ci concerne en effet un procédé de valorisation, sous la forme d'un ester, de l'acide acétique contenu dans le mélange de produits légers récupéré dans la fraction d'étêtage d'un procédé de purification de l'acide acrylique, ledit mélange contenant essentiellement de l'acide acétique, de l'acide acrylique, de l'acide formique et de l'eau, caractérisé par le fait que, dans un réacteur, on fait réagir ledit mélange avec un alcool choisi parmi les monoalcools primaires et secondaires, ayant un nombre d'atomes de carbone compris entre 4 et 8, en présence d'au moins un acide comme catalyseur, et d'au moins un inhibiteur de polymérisation, l'eau formée par cette réaction d'estérification étant entraînée continuellement par distillation, dans une colonne, sous la forme d'un mélange hétéroazéotropique avec l'alcool,
et par le fait que, lorsque les acides acétique, acrylique et formique ont été pratiquement totalement convertis en esters, on effectue les étapes de purification suivantes :
- dans une première étape, on fait réagir le mélange brut récupéré en fin de réaction d'estérification et une solution aqueuse basique, dans des conditions qui permettent de réaliser simultanément la neutralisation du catalyseur et des traces d'acides acétique, acrylique et formique résiduelles et la saponification sélective du formiate d'alkyle formé lors de la réaction d'estérification ;
- dans une deuxième étape, on sépare le mélange hétérogène obtenu, dans un décanteur, en une phase aqueuse inférieure qui est éliminée, et une phase organique supérieure ;
- dans une troisième étape, on distille la phase organique supérieure de l'étape précédente, dans une colonne, afin de récupérer, en tête, une fraction légère composée d'un mélange formé de l'alcool en excès et d'une partie de l'acétate d'alkyle, et, en pied, un mélange composé de l'acétate d'alkyle étêté et de produits lourds ;
- dans une quatrième étape, on distille, dans une colonne, le flux récupéré en pied de la colonne de la troisième étape, afin de récupérer, en tête, l'acétate d'alkyle pur, et, en pied, un mélange de composés lourds, formé principalement d'acrylate d'alkyle et d'une partie de l'acétate d'alkyle.

Le mélange de produits légers soumis à la réaction d'estérification comprend notamment 50 à 93% d'acide acétique, 1 à 30% d'acide acrylique, 0,5 à 5% d'acide formique, et 5 à 15% d'eau, ces pourcentages étant donnés en poids.

A l'étape d'estérification, on ajoute l'alcool, par exemple le butanol, dans un rapport molaire généralement de 1 à 2, en particulier de 1 à 1,3, à la somme des acides acétique, acrylique et formique présents dans le mélange des produits légers. On utilise, comme catalyseur, au moins un acide avantageusement choisi parmi l'acide sulfurique, l'acide méthane sulfonique et les acides benzène sulfoniques, ajouté(s) à raison de 0,05 à 2% en poids par rapport au mélange réactionnel, et, comme inhibiteur de polymérisation, de façon classiques, au moins un composé choisi parmi la phénothiazine, l'hydroquinone, l'éther méthylique de l'hydroquinone, ajouté(s) à raison de 0,05 à 0,15% en poids par rapport au mélange réactionnel.

L'étape d'estérification est généralement réalisée à une température comprise entre 80 et 140°C, préférentiellement à la température imposée par la composition du milieu sous pression atmosphérique.

Pendant la réaction d'estérification, on élimine continuellement l'eau formée par distillation dans une colonne en tête de laquelle on condense un mélange hétérogène azéotropique constitué principalement d'alcool et d'eau, puis on sépare une phase organique légère qui est renvoyée en tête de la colonne et une phase aqueuse lourde qui est éliminée.

A l'issue de l'étape de réaction d'estérification, lorsque la quasi-totalité des acides carboxyliques présents dans le milieu réactionnel initial a été convertie, on refroidit le mélange brut jusqu'à une température de 20 à 50°C.

On envoie dans ce brut une solution aqueuse alcaline diluée, laquelle peut être avantageusement obtenue par dilution d'une solution aqueuse plus concentrée à l'aide de la phase aqueuse lourde récupérée lors de l'étape d'estérification.

Pour cette opération de première étape de purification, on réalise simultanément la neutralisation des espèces acides présentes (catalyseur et éventuellement traces des acides carboxyliques qui n'ont pas réagi) et une saponification sélective du formiate d'alkyle qu'on élimine sous forme de sels alcalins dans la phase aqueuse après décantation. Pour éviter d'effectuer une saponification simultanée de l'acétate d'alkyle formé pendant l'estérification, on conduit cette opération à une température comprise entre 20 et 50°C, avec une solution basique diluée (soude) de concentration 2 à 8 N, un rapport molaire de base sur le formiate d'alkyle compris entre 1 et 5, de préférence entre 1,5 et 3, pendant une durée de 5 à 30 minutes. Le rendement de la réaction de saponification du formiate d'alkyle est supérieur à 99%.

Après élimination du formiate alcalin dans la phase aqueuse décantée de la réaction de saponification, le mélange brut résiduel est étêté dans une colonne à distiller, en tête de laquelle on récupère un mélange composé de l'alcool en excès et d'une partie de l'acétate d'alkyle, fraction qui peut être avantageusement recyclée à l'alimentation de l'étape d'estérification initiale. En pied de la colonne, on obtient un mélange constitué de l'acétate d'alkyle et des composés plus lourds, en particulier l'acrylate d'alkyle formé par estérification de l'acide acrylique présent initialement dans le milieu réactionnel.

Une dernière distillation de ce mélange permet de récupérer, en tête, l'acétate d'alkyle pur, et en pied, un mélange de lourds formés essentiellement de l'ester acrylique et d'une partie de l'acétate d'alkyle. Ces résidus lourds peuvent être distillés afin de récupérer une partie de l'acétate d'alkyle qui les compose.

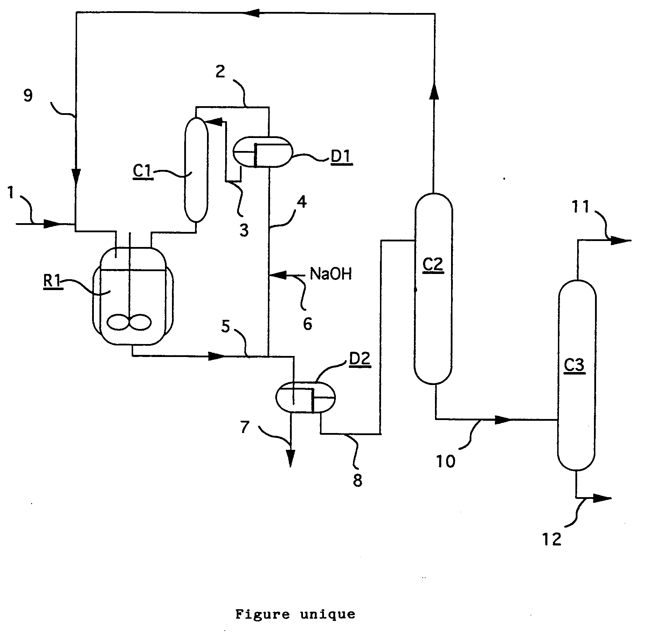

Le procédé qui vient d'être décrit peut être conduit en continu. Dans ce cas, si l'on se réfère à la Figure unique du dessin annexé, laquelle représente un schéma de ce procédé, on peut dire que ce dernier consiste à partir du mélange de légers récupérés dans la fraction d'étêtage d'un procédé de purification de l'acide acrylique, à réaliser dans un réacteur R1 une estérification des acides carboxyliques présents dans le mélange (acides acétique, acrylique et formique) par réaction dudit mélange et d'un alcool tel que défini ci-dessus, en présence d'un acide comme catalyseur et d'un inhibiteur de polymérisation (flux 1), l'eau formée par la réaction d'estérification étant entraînée par distillation dans une colonne C1 sous la forme d'un mélange hétéroazéotropique avec l'alcool, lequel mélange est soumis, après condensation (flux 2), à une séparation, dans un décanteur D1, pour donner une phase organique supérieure que l'on recycle en tête de colonne de distillation (flux 3), et une phase aqueuse inférieure que l'on soutire (flux 4).

Lorsque les acides carboxyliques sont totalement convertis en esters, on effectue les étapes de purification suivantes :
- dans une première étape, on réalise simultanément l'étape neutralisation - saponification décrite ci-dessus, en faisant réagir le mélange brut récupéré en fin de réaction d'estérification (flux 5) et une solution aqueuse basique, éventuellement préparée par dilution d'une solution aqueuse basique concentrée (flux 6) avec la phase aqueuse récupérée dans le décanteur D1 (flux 4),
- dans une deuxième étape, le mélange hétérogène obtenu est séparé, dans un décanteur D2, en une phase aqueuse inférieure (flux 7), laquelle est éliminée, et une phase organique supérieure (flux 8),
- dans une troisième étape, la phase organique de l'étape précédente est distillée dans une colonne C2, qui permet de récupérer en tête une fraction légère (flux 9), composée d'un mélange d'alcool et d'acétate d'alkyle, qui peut être recyclée à l'alimentation de l'étape initiale d'estérification, et en pied le mélange d'acétate d'alkyle étêté et de lourds (flux 10),
- dans une dernière étape, le flux récupéré en pied de colonne C2 est envoyé dans une colonne C3, qui permet de séparer en tête l'acétate d'alkyle pur (flux 11) et en pied un mélange composé principalement d'acrylate d'alkyle et d'acétate d'alkyle.

Le procédé selon l'invention peut également être conduit de façon discontinue ; à cet effet, on utilise une installation qui comprend un réacteur surmonté d'une colonne et d'un décanteur, et dans laquelle est d'abord conduite l'étape d'estérification, on conduit ensuite dans ledit réacteur les première et deuxième étapes de purification, respectivement de neutralisation - saponification et de décantation, puis on conduit les troisième et quatrième étapes de purification successivement dans ladite colonne, la fraction légère issue de la troisième étape étant conservée pour être recyclée au début de l'étape de réaction d'estérification du lot suivant, et les résidus lourds issus de la quatrième étape de plusieurs synthèses successives pouvant être rassemblés pour être distillés afin de récupérer une partie de l'acétate d'alkyle qui les compose.

Pour mieux illustrer l'objet de la présente invention, on va en décrire ci-après plusieurs exemples, non limitatifs. Dans ces exemples, l'effluent récupéré dans la fraction de tête du procédé de purification de l'acide acrylique présente la composition suivante, en poids : acide acétique (86,7%), acide formique (3,8%), acide acrylique (2,3%), eau (7,2%).

La réaction d'estérification et les étapes de purification sont réalisées en discontinu, dans un montage constitué par un réacteur de capacité 1 litre, équipé d'un agitateur, chauffé électriquement à l'aide d'un chauffe-réacteur, surmonté d'une colonne adiabatique à garnissage multiknitt, d'efficacité 12 plateaux théoriques. La colonne est elle-même équipée d'un condenseur et d'un décanteur.

### Exemple 1

Dans le montage décrit ci-dessus, on charge 374 g de fraction de tête du procédé de purification de l'acide acrylique, 449 g de n-butanol, 0,82 g d'acide sulfurique et 0,8 g de phénothiazine.

La température du mélange évolue, sous pression atmosphérique, entre 102°C (valeur initiale) et 123°C (valeur finale). A l'issue d'un temps de réaction de 75 minutes, une analyse indique une teneur en acides carboxyliques résiduels inférieure à 0,1% en poids. La phase aqueuse récupérée dans le décanteur représente 132 g. Le rendement de la réaction d'estérification de l'acide acétique est de 96%.

Le brut obtenu est refroidi jusqu'à 25°C, température à laquelle on ajoute une solution aqueuse de soude préparée par mélange de 51 g de soude à 49% avec 63 g de phase aqueuse récupérée lors de l'étape d'estérification. Après un temps de réaction de 20 minutes sous agitation, le mélange hétérogène est séparé en une phase aqueuse qui est éliminée et une phase organique (679 g) composée, en poids, de 88,7% d'acétate de butyle, 8,4% de butanol, 1,3% d'acrylate de butyle et 1,5% d'eau. On vérifie que la conversion du formiate de butyle en sel alcalin est totale.

Le montage est placé sous une pression de 9,3 x 10⁴ Pa (700 mmHg), et on distille une première fraction légère (129 g) composée, en poids, de 45,3% de butanol, 50,9% d'acétate butyle et 3,8% d'eau, puis une deuxième fraction (493 g) d'acétate de butyle, d'une pureté de 99%. Le résidu de la distillation représente 46 g, composé, en poids, de 19% d'acrylate de butyle et 79% d'acétate de butyle. Le rendement, exprimé en acétate de butyle purifié par rapport à l'acide acétique initial, est de 81%.

### Exemple 2

Une deuxième synthèse, puis une troisième synthèse sont réalisées, dans les mêmes conditions, hormis le fait qu'on introduit dans le réacteur la totalité de la fraction légère obtenue lors de la première fraction de distillation de la synthèse précédente, et qu'on complète le mélange réactionnel par les autres constituants (fraction de tête de distillation du procédé de synthèse d'acide acrylique, butanol, acide sulfurique et phénothiazine), de façon à obtenir les mêmes rapports entre chaque constituant. Les rendements de la réaction d'estérification de l'acide acétique sont respectivement de 99,5% et 99%. Après neutralisation des espèces acides et saponification simultanée du formiate de butyle, puis distillation de la fraction légère, et enfin de la fraction pure, les rendements d'acétate de butyle purifié par rapport à l'acide acétique initial sont de 97% pour chacune des deux synthèses successives. Le rendement global sur l'ensemble des trois opérations de synthèse est de 92%.

### Exemple 3

Dans le même montage que celui décrit ci-dessus, et dans des conditions de réaction identiques, on réalise l'estérification en présence d'acide méthane sulfonique, à une concentration massique de 0,05% par rapport au milieu réactionnel initial. La durée de réaction est de 90 minutes pour une conversion totale des acides carboxyliques présents (teneur résiduelle inférieure à 0,1% en poids).

### Exemple 4

Sur un brut réactionnel récupéré à l'issue de l'étape d'estérification, refroidi à 20°C on fait réagir une solution aqueuse de soude 2N, en quantité telle que le rapport molaire de la base sur le formiate de butyle présent, après neutralisation de toutes les espèces acides présentes, soit de 2.

Les rendements d'hydrolyse du formiate de butyle sont respectivement, pour des temps de réaction de 3 mn, 7 mn, 10 mn et 20 mn, de 76%, 96%, 98%, 99,5%. Corrélativement, le rendement de la réaction de saponification de l'acétate de butyle n'est que de 0,5% à l'issue de 20 minutes de réaction. Le rendement de cette réaction indésirable devient 3,4% lorsqu'un rapport molaire de soude (sous forme de soude 2N) au formiate de butyle de 4,3 est utilisé.

On récupère 10 g de la phase aqueuse de l'essai de saponification réalisé en 20 minutes avec rapport molaire de 2, et on la met en contact pendant un temps de 10 minutes, sous agitation, avec 7 g de résine sulfonique acide Amberlyst 15. Par cette opération, on régénère l'acide formique qui était sous forme de formiate de sodium dans la phase aqueuse. On vérifie par analyse qu'on récupère ainsi 98% de l'acide formique théorique présent dans le brut réactionnel sous forme de formiate de butyle.

## Revendications

1. Procédé de valorisation, sous la forme d'un ester, de l'acide acétique contenu dans le mélange de produits légers récupéré dans la fraction d'étêtage d'un procédé de purification de l'acide acrylique, ledit mélange contenant essentiellement de l'acide acétique, de l'acide acrylique, de l'acide formique et de l'eau, caractérisé par le fait que, dans un réacteur, on fait réagir ledit mélange avec un alcool choisi parmi les monoalcools primaires et secondaires, ayant un nombre d'atomes de carbone compris entre 4 et 8, en présence d'au moins un acide comme catalyseur, et d'au moins un inhibiteur de polymérisation, l'eau formée par cette réaction d'estérification étant entraînée continuellement par distillation, dans une colonne, sous la forme d'un mélange hétéroazéotropique avec l'alcool,
et par le fait que, lorsque les acides acétique, acrylique et formique ont été pratiquement totalement convertis en esters, on effectue les étapes de purification suivantes :
- dans une première étape, on fait réagir le mélange brut récupéré en fin de réaction d'estérification et une solution aqueuse basique, dans des conditions qui permettent de réaliser simultanément la neutralisation du catalyseur et des traces d'acides acétique, acrylique et formique résiduelles et la saponification sélective du formiate d'alkyle formé lors de la réaction d'estérification ;
- dans une deuxième étape, on sépare le mélange hétérogène obtenu, dans un décanteur, en une phase aqueuse inférieure qui est éliminée, et une phase organique supérieure ;
- dans une troisième étape, on distille la phase organique supérieure de l'étape précédente, dans une colonne, afin de récupérer, en tête, une fraction légère composée d'un mélange formé de l'alcool en excès et d'une partie de l'acétate d'alkyle, et, en pied, un mélange composé de l'acétate d'alkyle étêté et de produits lourds ;
- dans une quatrième étape, on distille, dans une colonne, le flux récupéré en pied de la colonne de la troisième étape, afin de récupérer, en tête, l'acétate d'alkyle pur, et, en pied, un mélange de composés lourds, formé principalement d'acrylate d'alkyle et d'une partie de l'acétate d'alkyle.

2. Procédé selon la revendication 1, caractérisé par le fait que le mélange de produits légers soumis à la réaction d'estérification comprend 50 à 93% d'acide acétique, 1 à 30% d'acide acrylique, 0,5 à 5% d'acide formique, et 5 à 15% d'eau, ces pourcentages étant donnés en poids.

3. Procédé selon l'une des revendications 1 et 2, caractérisé par le fait qu'à l'étape d'estérification, on ajoute l'alcool dans un rapport molaire de 1 à 2, en particulier, de 1 à 1,3, à la somme des acides acétique, acrylique et formique présents dans le mélange des produits légers.

4. Procédé selon l'une des revendications 1 à 3, caractérisé par le fait que l'on utilise, comme catalyseur, au moins un acide choisi parmi l'acide sulfurique, l'acide méthane sulfonique et les acides benzène sulfoniques, ajouté(s) à raison de 0,05 à 2% en poids par rapport au mélange réactionnel.

5. Procédé selon l'une des revendications 1 à 4, caractérisé par le fait que l'on conduit l'estérification à une température comprise entre 80 et 140°C.

6. Procédé selon l'une des revendications 1 à 5, caractérisé par le fait qu'en tête de la colonne surmontant le réacteur d'estérification, on condense le mélange hétéroazéotropique eau-alcool, et on le soumet à une séparation, dans un décanteur, pour donner une phase organique supérieure que l'on recycle en tête de la colonne de distillation, et une phase aqueuse inférieure que l'on soutire.

7. Procédé selon la revendication 6, caractérisé par le fait que l'on utilise la phase aqueuse inférieure soutirée du décanteur à la fin de l'étape d'estérification pour diluer une solution aqueuse basique concentrée, afin de former la solution aqueuse basique destinée à réagir avec le mélange brut d'estérification dans la première étape de saponification.

8. Procédé selon l'une des revendications 1 à 7, caractérisé par le fait que l'on conduit l'opération de la première étape de purification à une température comprise entre 20 et 50°C avec une solution basique diluée 2 à 8N, un rapport molaire de base sur le formiate d'alkyle compris entre 1 et 5, de préférence entre 1,5 et 3, pendant une durée de 5 à 30 minutes.

9. Procédé selon l'une des revendications 1 à 8, caractérisé par le fait que l'on recycle à l'alimentation de l'étape d'estérification initiale la fraction de tête issue de la colonne de distillation de la troisième étape de la purification.

10. Procédé selon l'une des revendications 1 à 9, caractérisé par le fait qu'on distille les résidus lourds issus de la quatrième étape de purification, afin de récupérer une partie de l'acétate d'alkyle qui les compose.

11. Procédé selon l'une des revendications 1 à 10, caractérisé par le fait qu'il est conduit en continu.

12. Procédé selon l'une des revendications 1 à 10, caractérisé par le fait qu'il est conduit de façon discontinue, et qu'on utilise à cet effet une installation qui comprend un réacteur surmonté d'une colonne et d'un décanteur, et dans laquelle est d'abord conduite l'étape d'estérification, que l'on conduit ensuite dans ledit réacteur les première et deuxième étapes de purification, respectivement de neutralisation - saponification et de décantation, puis que l'on conduit les troisième et quatrième étapes de purification successivement dans ladite colonne, la fraction légère issue de la troisième étape étant conservée pour être recyclée au début de l'étape de réaction d'estérification du lot suivant, et les résidus lourds issus de la quatrième étape de plusieurs synthèses successives pouvant être rassemblés pour être distillés afin de récupérer une partie de l'acétate d'alkyle qui les compose.

## Claims

1. Process for valorizing, in the form of an ester, the acetic acid contained in the mixture of light products recovered in the topping fraction of a process for the purification of acrylic acid, the said mixture essentially containing acetic acid, acrylic acid, formic acid and water, characterized in that, in a reactor, the said mixture is reacted with an alcohol chosen from primary and secondary monoalcohols having a number of carbon atoms of between 4 and 8, in the presence of at least one acid as catalyst and of at least one polymerization inhibitor, the water formed by this esterification reaction being continually entrained by distillation in a column in the form of a heteroazeotropic mixture with the alcohol,
and in that, when the acetic, acrylic and formic acids have been virtually completely converted to esters, the following purification stages are carried out:
- in a first stage, the crude mixture recovered at the end of the esterification reaction and a basic aqueous solution are reacted under conditions which make it possible to carry out simultaneously the neutralization of the catalyst and of the residual traces of acetic, acrylic and formic acids and the selective saponification of the alkyl formate formed during the esterification reaction;
- in a second stage, the heterogeneous mixture obtained is separated, in a settling tank, into a lower aqueous phase, which is discarded, and an upper organic phase;
- in a third stage, the upper organic phase from the preceding stage is distilled in a column, in order to recover, at the top, a light fraction composed of a mixture formed of the excess alcohol and of a portion of the alkyl acetate and, at the bottom, a mixture composed of the topped alkyl acetate and of heavy products;
- in a fourth stage, the flow recovered at the bottom of the column of the third stage is distilled in a column, in order to recover, at the top, the pure alkyl acetate and, at the bottom, a mixture of heavy compounds mainly formed of alkyl acrylate and of a portion of the alkyl acetate.

2. Process according to Claim 1, characterized in that the mixture of heavy products subjected to the esterification reaction comprises 50 to 93% of acetic acid, 1 to 30% of acrylic acid, 0.5 to 5% of formic acid and 5 to 15% of water, these percentages being given by weight.

3. Process according to either of Claims 1 and 2, characterized in that, in the esterification stage, the alcohol is added in a molar ratio of 1 to 2, in particular of 1 to 1.3, with respect to the sum of the acetic, acrylic and formic acids present in the mixture of the light products.

4. Process according to one of Claims 1 to 3, characterized in that use is made, as catalyst, of at least one acid chosen from sulphuric acid, methanesulphonic acid and benzenesulphonic acids, added in the proportion of 0.05 to 2% by weight with respect to the reaction mixture.

5. Process according to one of Claims 1 to 4, characterized in that the esterification is carried out at a temperature of between 80 and 140°C.

6. Process according to one of Claims 1 to 5, characterized in that, at the top of the column surmounting the esterification reactor, the water-alcohol heteroazeotropic mixture is condensed and is subjected to a separation, in a settling tank, in order to give an upper organic phase, which is recycled at the top of the distillation column, and a lower aqueous phase, which is withdrawn.

7. Process according to Claim 6, characterized in that use is made of the lower aqueous phase withdrawn from the settling tank at the end of the esterification stage to dilute a concentrated basic aqueous solution, in order to form the basic aqueous solution intended to react with the crude esterification mixture in the first saponification stage.

8. Process according to one of Claims 1 to 7, characterized in that the operation of the first purification stage is carried out at a temperature of between 20 and 50°C with a 2 to 8N dilute basic solution, a molar ratio of base to the alkyl formate of between 1 and 5, preferably between 1.5 and 3, for a time of 5 to 30 minutes.

9. Process according to one of Claims 1 to 8, characterized in that the top fraction resulting from the distillation column of the third stage of the purification is recycled to the feed of the initial esterification stage.

10. Process according to one of Claims 1 to 9, characterized in that the heavy residues resulting from the fourth purification stage are distilled, in order to recover a portion of the alkyl acetate of which they are composed.

11. Process according to one of Claims 1 to 10, characterized in that it is carried out continuously.

12. Process according to one of Claims 1 to 10, characterized in that it is carried out batchwise and that use is made, for this purpose, of a plant which comprises a reactor surmounted by a column and by a settling tank and in which the esterification stage is first carried out, that the first and second purification stages, respectively of neutralization-saponification and of separation by settling, are subsequently carried out in the said reactor, and then that the third and fourth purification stages are carried out successively in the said column, the light fraction resulting from the third stage being stored, in order to be recycled at the beginning of the esterification reaction stage of the following batch, and it being possible for the heavy residues resulting from the fourth stage of several successive syntheses to be combined, in order to be distilled in order to recover a portion of the alkyl acetate of which they are composed.

## Patentansprüche

1. Verfahren zur Nutzbarmachung der Essigsäure in Form eines Esters, die in dem Gemisch leichtsiedender Produkte enthalten ist, das in der Kopffraktion eines Verfahrens zur Reinigung von Acrylsäure gewonnen wird, wobei das Gemisch im wesentlichen Essigsäure, Acrylsäure, Ameisensäure und Wasser enthält,
dadurch gekennzeichnet, daß
das obige Gemisch in einem Reaktionsgefäß mit einem Alkohol, der unter primären und sekundären einwertigen Alkoholen mit 4 bis 8 Kohlenstoffatomen ausgewählt wird, in Gegenwart von mindestens einer als Katalysator dienenden Säure und mindestens eines Polymerisationsinhibitors umgesetzt wird, wobei das bei dieser Veresterungsreaktion gebildete Wasser kontinuierlich durch Destillation in einer Kolonne in Form eines heteroazeotropen Gemischs mit dem Alkohol entfernt wird,
und daß, wenn die Essigsäure, die Acrylsäure und die Ameisensäure praktisch vollständig in Ester umgewandelt worden sind, die folgenden Reinigungsschritte durchgeführt werden:
- in einem ersten Schritt wird das am Ende der Veresterungsreaktion gewonnene Rohgemisch mit einer wäßrigen basischen Lösung unter Bedingungen umgesetzt, die die gleichzeitige Neutralisation des Katalysators und der Spuren an restlicher Essigsäure, Acrylsäure und Ameisensäure sowie die selektive Verseifung des bei der Veresterungsreaktion gebildeten Alkylformiats ermöglichen;
- in einem zweiten Schritt wird das erhaltene heterogene Gemisch in einem Dekantiergefäß in eine untere wäßrige Phase, die entfernt wird, und eine obere organische Phase aufgetrennt;
- in einem dritten Schritt wird die obere organische Phase aus dem vorhergehenden Schritt in einer Kolonne destilliert, um am Kopf eine leichtsiedende Fraktion, die aus einem Gemisch aus überschüssigem Alkohol und einem Teil des Alkylacetats besteht und am Fuß ein Gemisch zu gewinnen, das aus nicht am Kopf übergegangenem Alkylacetat und schwersiedenden Produkten zusammengesetzt ist;
- in einem vierten Schritt wird der im dritten Schritt am Fuß der Kolonne gewonnene Fluß destilliert, um am Kopf das reine Alkylacetat und am Fuß ein Gemisch aus schwersiedenden Verbindungen, das im wesentlichen aus Alkylacrylat und einem Teil des Alkylacetats besteht, zu gewinnen.

2. Verfahren nach Anspruch 1, dadurch gekennzeichnet, daß das Gemisch aus leichtsiedenden Produkten, das der Veresterungsreaktion unterzogen wird, 50 bis 93 % Essigsäure, 1 bis 30 % Acrylsäure, 0,5 bis 5 % Ameisensäure und 5 bis 15 % Wasser enthält, wobei diese Prozentangaben gewichtsbezogen sind.

3. Verfahren nach einem der Ansprüche 1 und 2, dadurch gekennzeichnet, daß im Veresterungsschritt der Alkohol in einem Molverhältnis von 1 bis 2, insbesondere 1 bis 1,3, bezogen auf die Summe aus Essigsäure, Acrylsäure und Ameisensäure, die in dem Gemisch der leichtsiedenden Produkte enthalten sind, zugegeben wird.

4. Verfahren nach einem der Ansprüche 1 bis 3, dadurch gekennzeichnet, daß mindestens eine Säure als Katalysator verwendet wird, die unter Schwefelsäure, Methansulfonsäure und Benzolsulfonsäuren ausgewählt wird, wobei die Säure(n) in einem Anteil von 0,05 bis 2 Gew.-%, bezogen auf das Reaktionsgemisch, zugegeben wird/werden.

5. Verfahren nach einem der Ansprüche 1 bis 4, dadurch gekennzeichnet, daß die Veresterung bei einer Temperatur von 80 bis 140 °C durchgeführt wird.

6. Verfahren nach einem der Ansprüche 1 bis 5, dadurch gekennzeichnet, daß am Kopf der Kolonne oberhalb des Veresterungreaktionsgefäßes das heteroazeotrope Wasser-Alkohol-Gemisch kondensiert wird und einer Trennung in einem Dekantiergefäß unterzogen wird, um eine obere organische Phase, die am Kopf der Destillationskolonne wieder zugeführt wird, und eine untere wäßrige Phase, die abgelassen wird, zu erhalten.

7. Verfahren nach Anspruch 6, dadurch gekennzeichnet, daß die untere wäßrige Phase, die aus dem Dekantiergefäß abgelassen wird, am Ende des Veresterungsschritts zur Verdünnung einer konzentrierten wäßrigen basischen Lösung verwendet wird, um die wäßrige basische Lösung zu bilden, die für die Umsetzung mit dem rohen Veresterungsgemisch in dem ersten, aus der Verseifung bestehenden Schritt vorgesehen ist.

8. Verfahren nach einem der Ansprüche 1 bis 7, dadurch gekennzeichnet, daß der erste Reinigungsschritt bei einer Temperatur von 20 bis 50 °C mit einer basischen Lösung, die auf 2 bis 8 N verdünnt ist, bei einem Molverhältnis der Base zum Alkylformiat, das 1 bis 5, vorzugsweise 1,5 bis 3, beträgt während einer Dauer von 5 bis 30 min durchgeführt wird.

9. Verfahren nach einem der Ansprüche 1 bis 8, dadurch gekennzeichnet, daß die Kopffraktion, die von der Destillationskolonne des dritten Reinigungsschritts stammt, in die Einspeisung des anfänglichen Veresterungsschritts rückgeführt wird.

10. Verfahren nach einem der Ansprüche 1 bis 9, dadurch gekennzeichnet, daß die aus dem vierten Reinigungsschritt stammenden hochsiedenden Rückstände destilliert werden, um einen Teil des in den Rückständen enthaltenen Alkylacetats zu gewinnen.

11. Verfahren nach einem der Ansprüche 1 bis 10, dadurch gekennzeichnet, daß es kontinuierlich geführt wird.

12. Verfahren nach einem der Ansprüche 1 bis 10, dadurch gekennzeichnet, daß es diskontinuierlich geführt wird, daß hierzu eine Vorrichtung verwendet wird, die ein Reaktionsgefäß umfaßt, über dem eine Kolonne und ein Dekantiergefäß angeordnet sind, in der zunächst der Veresterungsschritt durchgeführt wird, daß anschließend in diesem Reaktionsgefäß der erste und der zweite Reinigungsschritt, d.h. der Neutralisations-Verseifungsschritt und der Dekantierschritt, durchgeführt werden, daß danach in dieser Kolonne nacheinander der dritte und der vierte Reinigungsschritt durchgeführt werden, wobei die aus dem dritten Schritt stammende leichtsiedende Fraktion aufbewahrt wird, um in den Anfang des aus der Veresterungsreaktion bestehenden Schritts der folgenden Charge rückgeführt zu werden, und wobei die aus dem vierten Schritt mehrerer aufeinanderfolgender Synthesen stammenden schwersiedenden Reste zusammengefaßt werden können, um für die Rückgewinnung eines Teils des Alkylacetats, das in diesen Resten enthalten ist, destilliert zu werden.
